# EUROPEAN PATENT APPLICATION

(11) **EP 2 777 770 A1**
(43) Date of publication of application: **17.09.2014**
(21) Application number: 13159405.3
(22) Date of filing: 15.03.2013
(51) Int. Cl.: A61Q 5/02, A61K 8/73, A61K 8/02

(54) **Cleansing cloth for cleansing hair**

(71) Applicant: The Procter & Gamble Company, Cincinnati, OH 45202 (US)
(72) Inventor: Schummer, Helga, 64397 Modautal (DE)
(74) Representative: Holmes, Rosalind

(57) **Abstract**

A cleansing cloth for cleansing hair. The cleansing cloth comprises a substrate comprising a fibrous material, and the cleansing cloth comprises a cleansing composition and the cleansing composition comprises at least about 20% absorber compound. The cleansing cloth comprises from about 0.2 to about 0.5 g/decimetre of the cleansing composition. Also: a pouch comprising the cleansing cloth; a method for cleansing hair comprising applying the cleansing cloth to hair; the use of the cleansing cloth for cleansing hair; a process for creating the cleansing cloth; and a kit comprising a substrate and a dry shampoo product.

## Description

### FIELD OF THE INVENTION

A cleansing cloth for cleansing hair, wherein the cleansing cloth comprises a substrate comprising a fibrous material, and wherein the cleansing cloth comprises a cleansing composition.

### BACKGROUND OF THE INVENTION

Dry shampoos are typically powder-based products where the powder is applied to the hair, allowed to absorb oil, particles e.g. dandruff, and sebum from the hair, and then the powder removed. Dry shampoos are typically used instead of usual surfactant-based rinse-out shampoo products which require wetting the hair. For example, where the consumer has no access to running water, or where he or she has no time to shower or bath, then a dry shampoo product may be used. Dry shampoos typically strip the hair of hair dye less than surfactant-based rinse-out shampoo products, even those taylored for use with coloured hair, thus can be employed on days between days where said rinse-out shampoos are used. This works to prolong vibrant hair colour. Other benefits of dry shampoos are: improved hair fragrance, reduced 'stringy' hair appearance, increased hair volume.

Most known dry shampoos comprise starch as the active ingredient. Examples of starches used include: corn starch, oryza sativa (rice) starch, zea mays starch, aluminium starch octenylsuccinate, solanum tuberosum starch, tapioca starch. Alcohol is also normally present, which is typically either isopropyl alcohol or ethanol. Other common ingredients include skin and/or hair conditioning agents, dyes, pH adjusters, preservatives, and fragrance. Some dry shampoos are packaged as aerosols others are available as straight powder. Typically, dry shampoos are packaged as aerosol products in tin-plated steel or aluminium spray cans comprising a liquified gas propellant such as butane, isobutane and/or propane. Non-aerosol forms of dry shampoo products are also available and these are normally packaged in plastics containers, jars, pots or tubes.

The inventor herein has, however, discovered numerous disadvantages to the products currently available on the market. These disadvantages can be summarised as follows: all traces of powder are difficult to remove from the hair and residues of powder normally remain - this is particularly unsightly for darker hair and causes the consumer concern that others will believe they have severe dandruff, eczema or another skin condition; the residues of remaining powder create a bad hair feel, hair dullness, appearance of unhealthy hair and lack of hair shine, and can cause scalp itchiness; when spraying the aerosol product or distributing non-spray products onto the hair, it is very difficult to avoid getting the powder on the face, in the eyes and onto clothing and the surrounding area e.g. bathroom furnishings - provisions to prevent this such as protecting the shoulders, clothes, and bathroom with e.g. towels are usually required; it is not easy to evenly apply the powder to the whole head of hair - the powder is usually not evenly distributed within the spray beam of aerosol products i.e. the spray pattern is typically not even, thus regular shaking and reshaking of the can is needed to improve this; the recommended means for removing all traces of the powder from the hair are typically not completely successful and normally a towel and/or a hairdryer are needed as well; spraying the aerosol products onto the scalp feels to certain consumers cold and unpleasant and, to some, feels like spraying underarm deodorant onto the hair - which unsurprisingly does not seem sensible/helpful; also some consumers have health concerns in relation to the spraying of small particles and the potential inhalation of powderous substances and their possible accumulation in the lungs.

Therefore, there is a need for a hair cleansing product that is: cleaner, easier and safer to apply, particularly with regards to obviating the need for protecting the shoulders, clothes and surrounding area from being contaminated with powder; no more need for using a towel or hairdryer to get the remaining powder from the hair; the ability to fit the product in your pocket or handbag i.e. a light, small, convenient and 'to go' product; reduced dulling of the hair and improved hair feel i.e. better hair look and feel; less contact of the product with the scalp such that any risk of scalp itchiness is lessened; reduced perceived risk of inhaling powderous substances; easier to distribute evenly across the head of hair. Also desired is for the product to provide improved hair volume and/or other hair health/style benefit(s). Also advantageous would be a product that is more environmentally friendly, sustainable and one that generates less waste.

### SUMMARY OF THE INVENTION

According to a first aspect, the present invention relates to a cleansing cloth for cleansing hair, wherein the cleansing cloth comprises a substrate comprising a fibrous material, and wherein the cleansing cloth comprises a cleansing composition, wherein the cleansing composition comprises at least about 20% absorber compound, and wherein the cleansing cloth is substantially dry and the cleansing composition is substantially dry, wherein the cleansing cloth comprises from about 0.2 to about 0.5 g/decimetre of the cleansing composition.

According to a second aspect, the present invention relates to a pouch comprising the cleansing cloth of the first aspect, wherein the pouch is air- and water-tight or capable of being closed such that when in a closed state, the pouch is air- and water-tight.

According to a third aspect, the present invention relates to a method for cleansing hair, comprising: applying the cleansing cloth, according to the first aspect, to hair; wherein the cleansing composition is applied to substantially dry hair and wherein the method does not comprise wetting the hair.

According to a fourth aspect, the present invention relates to the use of the cleansing cloth, according to the first aspect, for cleansing hair.

According to a fifth aspect, the present invention relates to a process for creating a cleansing cloth, wherein the process comprises providing a substrate comprising a fibrous material and then subsequently applying a formulation onto the substrate, such that the cleansing cloth comprises from about 0.2 to about 0.5 g/decimetre of the cleansing composition as described in the first aspect.

According to a sixth aspect, the present invention relates to a kit comprising: (a) a substrate comprising comprising a fibrous material, wherein the substrate is substantially dry and is substantially free of absorber compound; and (b) an aerosol dry shampoo product comprising a formulation, wherein the formulation in the aerosol dry shampoo product comprises: at least 5% absorber compound, from about 30% to about 50% alcohol, and from about 40% to about 60% propellant.

### DETAILED DESCRIPTION OF THE INVENTION

### General and definitions

In all embodiments of the present invention, all percentages are by weight of the total composition (or formulation), unless specifically stated otherwise. All ratios are weight ratios, unless specifically stated otherwise. All ranges are inclusive and combinable. The number of significant digits conveys neither a limitation on the indicated amounts nor on the accuracy of the measurements. All numerical amounts are understood to be modified by the word "about" unless otherwise specifically indicated. Unless otherwise indicated, all measurements are understood to be made at 25°C and at ambient conditions, where "ambient conditions" means conditions of about 1 atmosphere (atm) of pressure and at about 50% relative humidity. All such weights as they pertain to listed ingredients are based on the active level and do not include carriers or byproducts that may be included in commercially available materials, unless otherwise specified. "QS" or "QSP" means sufficient quantity for 100%. +/- indicates the standard deviation.

Herein, "comprising" means that other steps and other ingredients which do not affect the end result can be added. This term encompasses the terms "consisting of" and "consisting essentially of". The compositions, methods, uses, kits, and processes of the present invention can comprise, consist of, and consist essentially of the elements and limitations of the invention described herein, as well as any of the additional or optional ingredients, components, steps, or limitations described herein. The term "molecular weight" or "M.Wt." as used herein refers to the number average molecular weight unless otherwise stated.

Embodiments and aspects described herein may comprise or be combinable with elements or components of other embodiments and/or aspects despite not being expressly exemplified in combination, unless otherwise stated or an incompatibility is stated.

The term "aerosol" as used herein, means a suspension of fine droplets in a gas. An aerosol product atomises the composition in the can i.e. creates an aerosol. Due to surface tension, droplets are normally substantially spherical.

The term "substantially free from" or "substantially free of" as used herein means less than about 1%, less than about 0.8%, less than about 0.5%, less than about 0.3%, or about 0%, by total weight of the composition or formulation.

"Hair," as used herein, means mammalian hair including scalp hair, facial hair and body hair, more preferably hair on the human head and scalp. "Hair shaft" means an individual hair strand and may be used interchangeably with the term "hair."

"Cosmetically acceptable," as used herein, means that the compositions, formulations or components described are suitable for use in contact with human keratinous tissue without undue toxicity, incompatibility, instability, allergic response, and the like. All compositions and formulations described herein which have the purpose of being directly applied to keratinous tissue are limited to those being cosmetically acceptable.

"Derivatives," as used herein, includes but is not limited to, amide, ether, ester, amino, carboxyl, acetyl, acid, salt and/or alcohol derivatives of a given compound.

"Polymer," as used herein, means a chemical formed from the polymerisation of two or more monomers. The term "polymer" as used herein shall include all materials made by the polymerisation of monomers as well as natural polymers. Polymers made from only one type of monomer are called homopolymers. A polymer comprises at least two monomers. Polymers made from two or more different types of monomers are called copolymers. The distribution of the different monomers can be calculated statistically or block-wise - both possibilities are suitable for the present invention. Except if stated otherwise, the term "polymer" used herein includes any type of polymer including homopolymers and copolymers.

The term "hairstyling polymer" as used herein means hair-fixing polymers which form films on a surface i.e. film-forming polymers. In the context of hair, this surface is the surface of individual hair fibres or a plurality thereof. In the context of a fibrous material, it is the surface of the fibres. The hairstyling polymer causes the fibres to be glued together to build welds, which are cross-links that provide the hold benefit. In the context of a fibrous material, absorber compound can be given improved adherence to the substrate.

As used herein, the term "volatile organic compound" or "VOC", as used herein means any organic compound having a initial boiling point less than or equal to 250°C measured at a standard pressure of 101.3 kPa. In an embodiment, "VOC" means any compound having a vapour pressure of 0.01 kPa or more at 293.15 K (i.e. 20°C). "Organic" as used herein means any compound containing at least the element carbon and one or more of hydrogen, halogen, oxygen, sulfur, phosphorus, silicon, or nitrogen. Certain volatile compounds of organic chemistry falling within this definition are known to photochemically react with nitrogenic oxides in the presence of sunlight and, in turn, this produces ground-level ozone and photochemical smog. In fact, in the United States, the definition of VOC for US legislative purposes (U.S. EPA 40 CFR 51. 100[s]) defines only those organic compounds without negligible photochemical reactivity. Examples of compounds considered to be VOCs for the purposes of this application include: ethanol, dimethylether, 1,1-difluoroethane, 1,1,1,2-tetrafluoroethane, pentane, *n*-butane, *iso*-butane, propane, *trans-*1,3,3,3-tetrafluoropropene, free formic acid (i.e. not its salt). Certain fragrances and plant extracts are also VOCs.

"Separately packaged," as used herein, means any form of packaging that prevents one composition or formulation from coming into physical contact, or admixing, with a second composition or formulation.

"Kit," as used herein, means a packaging unit comprising a plurality of components i.e. a kit of parts. An example of a kit is, for example, a first composition and a separately packaged second composition. Another kit may comprise application instructions comprising a method and a composition/formulation.

"Dry" or "substantially dry" as used herein means comprising less than 5%, less than 3% or, less than 2%, less than 1%, or about 0% of any compound or composition being in liquid form when measured at 25°C at ambient conditions. Such compounds or compositions include water, oils, organic solvents and other wetting agents.

"Substantially" as used herein may mean at least about 50%, or at least about 60%, or at least about 70%, or at least about 80%, or at least about 80%, or at least about 90%.

"Anhydrous" as used herein means that the composition comprises less than 5%, less than 3% or, less than 2%, less than 1%, or about 0% water by total weight of the composition.

### Explanation of the improvement

The present invention relates to a cleansing cloth for cleansing hair as described herein. The cleansing cloth comprises a substrate comprising a fibrous material, and the cleansing cloth comprises a cleansing composition and the cleansing composition comprises at least about 20% absorber compound. The cleansing cloth comprises from about 0.2 to about 0.5 g/decimetre of the cleansing composition. Also provided is: a pouch comprising the cleansing cloth; a method for cleansing hair comprising applying the cleansing cloth to hair; the use of the cleansing cloth for cleansing hair; a process for creating the cleansing cloth; and a kit comprising a substrate and a dry shampoo product. These are as described herein.

The present invention provides improved hair cleansing versus the prior art, for example versus aerosol or non-aerosol dry shampoos. WO/02080727A discloses the use of cosmetic towels for styling hair, said towels being impregnated with a solution containing (a) film-forming media and (b) cationic surfactants. However, there is no disclosure in WO/02080727A of a cleansing cloth for cleansing hair, particularly no disclosure of a cloth similar to that of the present invention.

The benefits of the present invention include a way of hair cleansing that is cleaner, easier and safer to apply, particularly with regards to obviating the need for protecting the shoulders, clothes and surrounding area from being contaminated with powder. Indeed, the cleansing cloth comprises a substrate for the cleansing composition comprising the absorber and there is no need for spraying dry shampoo into the air or onto the hair. There is also now no need to use a towel or hairdryer to get the remaining powder from the hair - the cleansing cloth does not allow for over-application of the cleansing composition on the hair since the cleansing composition comprising absorber is already on the cleansing cloth in an appropriate amount. This has a great advantage over the prior art methods since it is very difficult to provide optimal on-pack instructions on convention dry shampoo products since i) there is very little space available for this e.g. on the aerosol can surface itself and ii) the optimal amount of dry shampoo that should be applied to the hair depends on many factors - to list a few: hair type, hair oiliness/dirtiness, density of hairs, colour of hair etc. Therefore, the consumer is left to experiment by trial and error as to how much dry shampoo product to apply that fits his or her needs and his or her hair's needs. Furthermore, where the consumer sprays too much dry shampoo onto the hair, then often he/she is forced to shower using a conventional rinse-off shampoo anyway, thus defeating the objective of using the dry shampoo in the first place. With the cleansing cloth pursuant to the present invention, the consumer both applies absorber to the hair and removes absorber from the hair concurrently by use of the present cloth. This way, the consumer sees instant results and thus receives instant feedback vis-à-vis how many more passages over the head of hair are needed in order to achieve the required look. The present invention is employs a specific type of substrate and amount of absorber such that it is very difficult for the consumer to over-use the cleansing cloth e.g. apply too much cleansing composition to the hair. Excellent results are achieved when the consumer employs the cleansing cloth in the same way he or she would use a towel to dry their hair after showering, thus use of the cleansing cloth does not require special training or instruction. The cleansing cloth of the present invention, by the action of the absorber to collect excess oils, particles and dirt from the hair, cleanses the hair providing reduced dulling of the hair and improved hair feel i.e. better hair shine, look and feel. Oily hair tends to clump together, thus the cloth of the present invention provide improved hair volume. Since the cleansing composition comprising absorber is already incorporated on/in the cleansing cloth, and the cleansing cloth is applied to the hair directly, less contact of the absorber with the scalp occurs such that any risk of scalp itchiness is lessened. Furthermore, there is no need to spray absorber into the air and therefore there is a highly reduced concern of inhaling powderous/particulate substances. Another important advantage of the present invention is the ability to fit the product, for example when it is in the form of a pouch comprising the cleansing cloth, in your pocket or handbag i.e. it is a light, small, convenient and 'to go' product. There is no need to carry around a large, bulky spray can and that the cleansing cloth comprises almost no VOC. Another important advantage is that the cleansing cloth allows for much more even distribution of the absorber compound over the head of hair, so that the maximum amount of fat and other hair 'dirt' can be taken up by the absorber. The fibrous material of the substrate of the cleansing cloth may also be a natural material such as cotton, which have high tolerances by consumers from an allergic standpoint and are preferred by consumers from a sustainability perspective. Also advantageous is a that it is more environmentally friendly, sustainable and generates less waste.

### 1^{st} aspect: Cloth

The first aspect relates to a cleansing cloth for cleansing hair as described herein. In an embodiment, the hair is human hair.

### Substrate

The cleansing cloth comprises a substrate comprising a fibrous material. In an embodiment, the fibrous material is selected from the group consisting of: plant-derived fibres; animal fur-derived fibres; mineral-derived fibres; and combinations thereof. In an embodiment, the cleansing cloth comprises a substrate comprising at least about 75%, or at least about 80%, or at least about 85%, or at least about 90%, or at least about 95% natural fibres. In an embodiment, natural fibres is any fibre being wool, cotton, flax, hemp, jute, kapok, nettle, kenaf, moss, bamboo, and combinations thereof. IN an embodiment, the natural fibres is wool, and wherein the wool is from sheep, angora, cashmere, mohair, alpaca, camel, and combinations thereof.

In an embodiment, the cleansing cloth comprises a substrate comprising a material selected from the group consisting of: synthetic fibres made from fibres of natural origin; synthetic fibres made from fibres of synthetic origin; and combinations thereof. In an embodiment, the substrate is viscose. In an embodiment, the substrate comprises viscose. Viscose is a synthetic fibre made from cellulose. In an embodiment, the substrate comprises at least about 80%, or at least about 85%, or at least about 90%, or at least about 95% casein fibres. Casein fibres can be made from milk. In an embodiment, the substrate comprises at least about 80%, or at least about 85%, or at least about 90%, or at least about 95% soya fibres. Soya fibres can be made from soya beans.

In an embodiment, the cleansing cloth comprises a substrate comprising a material selected from the group consisting of: non-woven; fleece; felt; knit; crochet; molleton; and combinations thereof. As used herein, "molleton" means the German material "Molton", which is a woven material, typically cotton, where the surface of the weave has been roughened. Molleton can also be made from other fibrous materials and not just cotton e.g. polyester.

In an embodiment, the natural fibre is cotton. In an embodiment, the substrate comprises at least about 75%, or at least about 80%, or at least about 85%, or at least about 90%, or at least about 95% cotton. In an embodiment, the substrate comprises at least about 75%, or at least about 80%, or at least about 85%, or at least about 90%, or at least about 95% cellulose. In an embodiment, the substrate is molleton and comprises at least about 95% cotton. In an embodiment, the substrate is molleton, wherein the substrate comprises least about 85%, or at least about 90%, or at least about 95% cotton. Cotton is useful in view of the structure of the individual fibres, which do not have a simple, regular oval or circular cross-section, but comprise a groove, usually two grooves down their lengths. This groove increases the surface area of each individual fibre and also provides a 'sheltered' place for the absorber compound to reside when in contact with the substrate. The groove is particularly suitable for absorber compounds being starch compounds in view of the compatibility of the starch compound with the shape, charge and chemistry of this groove. Cotton is also particularly preferred in view of the health benefits associated with this fibrous material, for example, reduced likelihood of irritation to the scalp versus synthetic fibrous materials. Cotton is also both lipophilic and hydrophilic, which is important for adhering absorber compounds comprising different types of 'dirt'.

In an embodiment, the fibrous material is a synthetic fibrous material. In an embodiment, the cleansing cloth comprises less than 5%, or less than 10%, or less than 20%, or less than 30%, or less than 50%, or less than 75% synthetic fibrous materials. In an embodiment, the synthetic fibrous materials are selected from the group consisting of: polyester, polyamide, polyimide, viscose, polyvinylchloride, acrylic, and combinations thereof.

In an embodiment, the substrate is a non-woven or fleece.

In an embodiment, the cleansing cloth is substantially flat. In an embodiment, the cleansing cloth is substantially flat and comprises a first face and a second face, wherein at least one of the faces comprises the fibrous material, wherein the fibrous material is on at least 50% of the surface of the face comprising the fibrous material (percentage is calculated by total surface area of the face).

In an embodiment, the fibrous material comprises fibres having an average dtex. Dtex is a unit of measure for the linear mass density of fibers and is defined as the mass in grams per 10,000 metres. In an embodiment, the fibrous material comprises fibres having an average dtex of 5 dtex or less, or 4 dtex or less, or 3 dtex or less, or 2 dtex or less, or 1 dtex or less, or 0.9 dtex or less, or 0.8 dtex or less, or 0.7 dtex or less, or 0.6 dtex or less, or 0.5 dtex or less. In an embodiment, at least 90% of the fibre in the fibrous material have an average dtex of 5 dtex or less, or 4 dtex or less, or 3 dtex or less, or 2 dtex or less, or 1 dtex or less, or 0.9 dtex or less, or 0.8 dtex or less, or 0.7 dtex or less, or 0.6 dtex or less, or 0.5 dtex or less.

In an embodiment, the cleansing cloth is substantially flat and comprises a first face and a second face, wherein the first face comprises the cleansing composition and the second face is substantially free of cleansing composition. In an embodiment, the first face comprises the cleansing composition and the second face is substantially free of cleansing composition, and wherein the first face further comprises a hairstyling polymer. The hairstyling polymer can act to help adhere the absorber present in the cleansing composition to the substrate. In an embodiment, the first face comprises the cleansing composition and the second face comprises a hair conditioning agent and/or a hairstyling polymer. Thus, the consumer can employ the first face comprising the cleansing composition to cleanse the hair, and then the second face to condition and/or style the hair. The second face, by being substantially free of cleansing composition in certain embodiments, may also be employed to help remove remaining cleansing composition from the hair. The descriptions of hair conditioning agents and hair styling polymers described below are suitable for describing this embodiment also.

In an embodiment, the cleansing cloth has a maximum length, wherein the maximum length is the longest dimension, of about 10 to about 60 cm, or from about 20 cm to about 45 cm, or from about 30 cm to about 40 cm.

In an embodiment, the cleansing cloth comprises a substrate having two layers. The cleansing composition may be sandwiched between the two layers and wherein the outer faces of each layer are substantially free of cleansing composition. In an embodiment, the cleansing cloth comprises a substrate having two layers, and wherein the cleansing composition may be sandwiched between the two layers, and wherein one layer comprises holes such that the cleansing composition can pass through the holes in order to reach the hair.

In an embodiment, the cleansing cloth is in a shape complementary to and capable of receiving a member selected from the group consisting of: a human hand or human a finger, a comb, a hair brush, a straightening or curling implement, and combinations thereof; or wherein the cloth is a glove for a human hand or human finger.

In an embodiment, the cleansing cloth is substantially flat and wherein the cleansing cloth has an area (length multiplied by width) of from about 100 cm² to about 3600 cm².

### Cleansing Composition

The cleansing cloth comprises from about 0.2 to about 0.5 g/decimetre of the cleansing composition. In an embodiment, the cleansing cloth comprises from about 0.21 to about 0.45 g/decimetre, or from about 0.21 to about 0.42 g/decimetre, or from about 0.22 to about 0.40 g/decimetre of the cleansing composition.

The cleansing composition comprises at least about 20% absorber compound. The absorber compound is important since it adheres fat molecules, dead skin particles and other material that makes hair look dirty and thus the absorber compound must be suitable for this purpose. Upon removal of the cleansing cloth from the hair following use, these 'dirt' materials are removed with the cleansing cloth. In an embodiment, the cleansing composition comprises at least about 25%, or at least about 30%, or at least about 35%, or at least about 40%, or at least about 45%, or at least about 50%, or at least about 55%, or at least about 60%, or at least about 65%, or at least about 70%, or at least about 75%, or at least about 80%, or at least about 85%, or at least about 90%, or at least about 95% absorber compound.

In an embodiment, the absorber compound is a starch compound. Starch compounds are useful in that they are based on a natural polysaccharide, which is also a renewable material. In an embodiment, the absorber compound is a starch compound and is selected from the group consisting of: oryza sativa (rice) starch, dimethylimidazolidinone rice starch, aluminium starch octenylsuccinate, corn starch, modified corn starch, zea mays starch, solanum tuberosum starch, tapioca starch, and mixtures thereof. In an embodiment, the starch compound is aluminium starch octenylsuccinate. In an embodiment, wherein the absorber compound is a starch compound and wherein the starch compound is aluminium starch octenylsuccinate. In an embodiment, the absorber compound is selected from the group consisting of: flour; clay; baking powder; sodium bicarbonate; agar; silica; talcum powder; and mixtures thereof.

In an embodiment, the cleansing composition is substantially free of any volatile organic compound. Volatile organic compounds are typically not desired since many consumers have health concerns vis-à-vis these compounds and stricter regulations exist in their amounts in products offered for sale as a result of these concerns. In an embodiment, the cleansing composition is substantially free of: castor oil; alcohol.

In an embodiment, the cleansing composition further comprises an additive selected from the group consisting of: glitter, particles, a preservative, a dye or pigment, skin or hair conditioning agent, pH adjuster, buffering agent, fragrance, panthenol, calendula, retinyl palmitate, bromelain, calcium panthotenate, amodimethicone, cyclopentasiloxane, silicone compound, mica, titanium dioxide, iron oxide, ascorbic acid, aminomethyl propanol, adipic acid, disodium phosphate, methyl- or propyl parabene, DMDM hydantoin, diazolidinyl urea, geraniol, amyl cinnamal, hexyl cinnamal, lemon grass oil, ascorbyl palmitate, and mixtures thereof. In an embodiment, the cleansing composition comprises glitter. Glitter is useful in that it gives a shiny appearance to the hair. In an embodiment, the glitter is coloured.

In an embodiment, the cleansing composition comprises a skin conditioning agent. In an embodiment, the skin conditioning agent is selected from the group consisting of: panthenol; calendula; retinyl palmitate; bromelain; and mixtures thereof. In an embodiment, the cleansing composition comprises from about 0.001% to about 5%, or from about 0.1% to about 4%, or from about 0.1% to about 3% of skin conditioning agent.

In an embodiment, the cleansing composition comprises an anti-dandruff agent. Anti-dandruff agents are useful in the cleansing composition in that they help reduce the fungus that contributes to dandruff - thus the user of the cleansing cloth can simultaneously cleanse their hair and also help prevent dandruff i.e. help prevent their hair from accumulating more 'dirt'. In an embodiment, the cleansing composition comprises from about 0.01% to about 5%, or from about 0.1% to about 4%, or from about 0.1% to about 3 % of anti-dandruff agent. In an embodiment, the anti-dandruff agent is selected from the group consisting of: zinc pyrithione, octopirox, climbazole, ketoconazole, and mixture thereof. The full chemical name of climbazole is 1-imidazolyl-1-(4-chlorophenoxy)-3,3-dimethylbutan-2-one.

In an embodiment, the cleansing composition comprises a hair conditioning agent. Hair conditioning agents are useful in the cleansing composition because they allow the user to condition their hair at the same time as cleansing it - similar to a conventional surfactant-based conditioning shampoo. In an embodiment, the hair conditioning agent is selected from the group consisting of: calcium panthotenate; amodimethicone; quaternary ammonium cationic surfactants; aminosilicone; cyclopentasiloxane; silicone quaternium compounds; and mixtures thereof. In an embodiment, the hair conditioning agent is quaternary ammonium cationic surfactant and wherein the quaternary ammonium cationic surfactant is selected from the group consisting of: cetyltrimethylammonium chloride, behenyltrimethylammonium chloride (BTAC), cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, cocotrimethylammonium chloride, dipalmitoylethyldimethylammonium chloride, PEG-2 oleylammonium chloride, and mixtures thereof. Also suitable are salts of these quaternary ammonium cationic surfactants, where the chloride is replaced by halogen (e.g., bromide), acetate, citrate, lactate, glycolate, phosphate nitrate, sulphate, or alkylsulphate.

In an embodiment, the cleansing composition comprises a triglyceride oil. In an embodiment, the triglyceride oil is selected from the group consisting of: avocado oil, olive oil, corn oil, rape seed oil, sesame oil, wheat germ oil, castor oil, linseed oil, sunflower oil, cottonseed oil, soybean oil, peanut oil, and mixtures thereof. In an embodiment, the cleansing composition comprises from about 0.01% to about 5%, or from about 0.1% to about 4%, or from about 0.2% to about 3%, or from about 0.2% to about 0.5% of triglyceride oil.

In an embodiment, the cleansing composition comprises a pigment or dye. In an embodiment, the pigment or dye is selected from the group consisting of: mica; titanium dioxide; iron oxide; and mixtures thereof. In an embodiment, the absorber compound exhibits a colour substantially corresponding to the colour of human hair. In an embodiment, both the cleansing cloth and the absorber compound exhibit a colour substantially corresponding to the colour of human hair. Human hair is naturally typically brown, grey, blonde, red, ginger, or black in colour. Human hair may be dyed such that this list of colours then also includes purple, blue, pink, green and the like.

In an embodiment, the composition comprises a pH adjuster. In an embodiment, the pH adjuster is selected from the group consisting of: ascorbic acid; aminomethyl propanol; adipic acid; disodium phophate; and mixtures thereof. In an embodiment, the composition comprises a preservative. In an embodiment, the preservative is selected from the group consisting of: methyl paraben; propyl paraben; DMDM hydantoin; diazolidinyl urea; and mixtures thereof. In an embodiment, the composition comprises a fragrance. In an embodiment, the fragrance is selected from the group consisting of: geraniol; amyl cinnamal; hexyl cinnamal; lemon grass oil; ascorbyl palmitate; and mixtures thereof.

In an embodiment, the cleansing composition further comprises a hairstyling polymer. In an embodiment, the hairstyling polymer is selected from the group consisting of: amphoteric hairstyling polymers, zwitterionic hairstyling polymers, anionic hairstyling polymers, non-ionic hairstyling polymers, cationic hairstyling polymers, and mixtures thereof. In an embodiment, the cleansing composition comprises from about 0.01 % to about 20%, or from about 0.01 % to about 16%, or from about 0.01 % to about 10%, or from about 1% to about 8%, or from about 2% to about 6% of a hairstyling polymer.

In an embodiment, the hairstyling polymer is a water-compatible hairstyling polymer, alternatively a water-soluble hairstyling polymer. In an embodiment, the cleansing composition is substantially free of a water-incompatible hairstyling polymer. An example of a water-incompatible hairstyling polymer includes an Acrylates/t-Butylacrylamide Copolymer which is a copolymer of tert-butyl acrylamide and one or more monomers of acrylic acid, methacrylic acid, or one of their simple esters (e.g. Ultrahold^{®} 8 from BASF). Balance^{®} CR from Akzo Nobel, which is an acrylates copolymer of two or more monomers of (meth)acrylic acid or one of their simple esters, is water-compatible. The octylacrylamide/acrylate/butylaminoethyl methacrylate copolymer Amphomer^{®} is also water-compatible. In an embodiment, the hairstyling polymer is a latex hairstyling polymer.

The cleansing composition may comprise a cationic hairstyling polymer. In an embodiment, the cationic hairstyling polymers is selected from group consisting of those with primary, secondary, tertiary or quaternary amino groups. In an embodiment, the cationic hairstyling polymer has a cationic charge density, and wherein the cationic charge density is from 1 to 7 meq/g. In an embodiment, the cationic hairstyling polymer comprises quaternary amino groups. In an embodiment, the cationic hairstyling polymer is a homo- or copolymer where the quaternary nitrogen groups are contained either in the polymer chain or as substituents on one or more of the monomers. The ammonium group-containing monomers can be copolymerized with non-cationic monomers. In an embodiment, the cationic hairstyling polymer comprises cationic monomers where the cationic monomers are unsaturated compounds that can undergo radical polymerization and which bear at least one cationic group. In an embodiment, the cationic monomers are selected from the group consisting of: ammonium-substituted vinyl monomers such as, for example, trialkylmethacryloxyalkylammonium, trialkylacryloxyalkylammonium, dialkyldiallylammonium and quaternary vinylammonium monomers with cyclic, cationic nitrogen-containing groups such as pyridinium, imidazolium or quaternary pyrrolidones, e.g. alkylvinylimidazolium, alkylvinylpyridinium, or alkylvinylpyrrolidone salts. The alkyl groups of these monomers may be lower alkyl groups such, as for example, C-1 to C-7 alkyl groups, and may also be are C-1 to C-3 alkyl groups.

In an embodiment, cationic hairstyling polymer comprises ammonium group-containing monomers being copolymerized with non-cationic monomers. The non-cationic monomers may be selected from the group consisting of: acrylamide, methacrylamide, alkyl- and dialkylacrylamide, alkyl- and dialkylmethacrylamide, alkyl acrylate, alkyl methacrylate, vinylcaprolactone, vinylcaprolactam, vinylpyrrolidone, vinyl esters, for example vinyl acetate, vinyl alcohol, propylene glycol or ethylene glycol, and mixture thereof. The alkyl groups of these monomers may be C-1 to C-7 alkyl groups, and may be C-1 to C-3 alkyl groups. In an embodiment, cationic hairstyling polymer comprises at least one quaternary amino group. Suitable polymers with at least one quaternary amino group include, for example, those described in the CTFA Cosmetic Ingredient Dictionary under the designations 'polyquaternium' such as methylvinylimidazolium chloride/vinylpyrrolidone copolymer (polyquaternium-16) or quaternized vinylpyrrolidone/dimethylaminoethyl methacrylate copolymer (polyquaternium-11; Gafquat^{®} 755N-PW from ISP) as well as quaternary silicone polymers or silicone oligomers such as, for example, silicone polymers with quaternary end groups (quatemium-80).

In an embodiment, the hairstyling polymer is a cationic hairstyling polymer being of synthetic origin. In an embodiment, the cationic hairstyling polymers of synthetic origin are selected from the group consisting of: poly(dimethyldiallylammonium chloride); copolymers from acrylamide and dimethyldiallylammonium chloride; quaternary ammonium polymers, formed by the reaction of diethyl sulfate with a copolymer from vinylpyrrolidone and dimethylaminoethyl methacrylate, especially vinylpyrrolidone/dimethylaminoethyl methacrylate methosulfate copolymer (e.g. Gafquat® 755 N; Gafquat^{®} 734); quaternary ammonium polymers from methylvinylimidazolium chloride and vinylpyrrolidone (e.g. Luviquat^{®} HM 550 from BASF; Luviquat^{®} Hold from BASF; polyquaternium-46 [vinylcaprolactam {VCap}, vinylpyrrolidone {VP} and quaternized vinylimidazole {QVI}] from BASF; Luviquat^{®} FC 905 from BASF [polyquaternium-16]); Luviquat Supreme^{®} from BASF (polyquaternium-68, quaternised copolymer of vinyl pyrrolidone, methacrylamides, vinyl imidazole and quaternized vinyl imidazole); polyquaternium-35; polyquaternium-57; polymers from trimethylammonium ethyl methacrylate chloride; terpolymers from dimethyldiallylammonium chloride, sodium acrylate and acrylamide (e.g. Merquat^{®} Plus 3300); copolymers from vinylpyrrolidone, dimethylaminopropyl methacrylamide, and methacryloylaminopropyllauryldimethylammonium chloride; terpolymers from vinylpyrrolidone, dimethylaminoethyl methacrylate, and vinylcaprolactam (e.g. Gaffix^{®} VC 713); vinylpyrrolidone/methacrylamidopropyltrimethylammonium chloride copolymers (e.g. Gafquat® HS 100); copolymers from vinylpyrrolidone and dimethylaminoethyl methacrylate; copolymers from vinylpyrrolidone, vinylcaprolactam, and dimethylaminopropylacrylamide; poly- or oligoesters formed from at least one first type of monomer that is selected from hydroxyacids substituted with at least one quaternary ammonium group; dimethylpolysiloxane substituted with quaternary ammonium groups in the terminal positions; and mixtures thereof.

In an embodiment, the hairstyling polymer is a cationic hairstyling polymer being of natural origin. In an embodiment, the cationic hairstyling polymers being of natural origin are selected from the group consisting of: cationic derivatives of polysaccharides, for example, cationic cellulose derivatives, starch, guar, and mixtures thereof. Cationic derivatives of polysaccharides may be represented by the general formula:

G-O-B-N⁺-R^{a}-R^{b}-R^{c} X⁻

G is an anhydroglucose residue, for example, starch or cellulose anhydroglucoses;
B is a divalent bonding group, for example, alkylene, oxyalkylene, polyoxyalkylene or hydroxyalkylene;
R^{a}, R^{b} and R^{c} are, independently from one another, alkyl, aryl, alkylaryl, arylalkyl, alkoxyalkyl or alkoxyaryl, any of which can have up to 22 carbon atoms, wherein the total number of carbon atoms in R^{a}, R^{b} and R^{c} is may be a maximum of 20.

In an embodiment, the hairstyling polymer is a cationic cellulose derivative being selected from the group consisting of: those that have at least one quaternary ammonium group, e.g. a copolymer made of hydroxyethyl cellulose and diallyldimethyl ammonium chloride (polyquaternium-4), or the reaction product made of hydroxyethyl cellulose and an epoxide substituted with a trialkyl ammonium group (polyquaternium-10), wherein the alkyl groups can have 1 to 20 carbon atoms, or wherein the alkyl group is methyl. In an embodiment, the hairstyling polymer is a cationic cellulose derivative having a molecular weight of from about 100,000 Da to about 600,000 Da, or from about 200,000 Da to about 400,000 Da. In an embodiment, the cationic cellulose derivative has a nitrogen content, wherein the nitrogen content is from about 0.5% to about 4%, or from about 1.5% to about 3%. In an embodiment, the hairstyling polymer is a cationic cellulose derivative being polyquaternium-4. Polyquaternium-4 is sold under the trade names Celquat® HlOO and Celquat^{®} L200, of which Celquat^{®} L200 is especially preferred.

In an embodiment, the hairstyling polymer is a cationic latex hairstyling polymer. In an embodiment, the cationic hairstyling polymer is selected from the group consisting of: polyquaternium-4, polyquaternium-11, polyquaternium-16, polyquaternium-68, mixtures thereof, and mixtures of polyquaternium-68 with a non-ionic hairstyling polymer. In an embodiment, the hairstyling polymer is selected from the group consisting of: polyquaternium-4, polyquaternium-11, polyquaternium-68, and mixtures thereof. In an embodiment, the cleansing composition comprises a chitosan, a chitosan salt or a chitosan derivative. In an embodiment, the cleansing composition comprises less than 0.1% by weight chitosan, chitosan salts and chitosan derivatives. In another embodiment, the cleansing composition is substantially free from chitosan, chitosan salts and chitosan derivatives. In an embodiment, the cleansing composition comprises a hairstyling polymer selected from the group consisting of: polyquaternium-4, polyquaternium-11, polyquaternium-16, polyquaternium-68, mixtures thereof; or from the group consisting of: polyquaternium-4, polyquaternium-68, and mixtures thereof. In an embodiment, the cleansing composition comprises a hairstyling polymer selected from the group consisting of: polyquaternium-4, polyquaternium-11, polyquaternium-68, mixtures thereof; or from the group consisting of: polyquaternium-4, polyquaternium-68, and mixtures thereof.

In an embodiment, the cleansing composition comprises less than about 0.5 wt% of a cationic hairstyling polymer by total weight of the cleansing composition.

In an embodiment, the cleansing composition comprises an amphoteric or zwitterionic hairstyling polymer. In an embodiment, the amphoteric or zwitterionic hairstyling polymer is selected from the group consisting of: copolymers formed from alkylacrylamide, alkylaminoalkyl methacrylate, and two or more monomers from acrylic acid and methacrylic acid as well as, if necessary, their esters, especially copolymers from octylacrylamide, acrylic acid, butylaminoethyl methacrylate, methyl methacrylate and hydroxypropyl methacrylate (octylacrylamide/acrylate/butylaminoethyl methacrylate copolymer, for example Amphomer^{®} from Akzo Nobel); copolymers, that are formed from at least one of a first type of monomer that possesses quaternary amino groups and at least one of a second type of monomer that possesses acid groups; copolymers from fatty alcohol acrylates, alkylamine oxide methacrylate and at least one monomer selected from acrylic acid and methacrylic acid as well as, if necessary, acrylic acid esters and methacrylic acid esters, especially copolymers from lauryl acrylate, stearyl acrylate, ethylamine oxide methacrylate and at least one monomer selected from acrylic acid and methacrylic acid as well as, if necessary, their esters; copolymers from methacryloyl ethyl betaine and at least one monomer selected from methacrylic acid and methacrylic acid esters; copolymers from acrylic acid, methyl acrylate and methacrylamidopropyltrimethylammonium chloride (polyquaternium-47); copolymers from acrylamidopropyltrimethylammonium chloride and acrylates or copolymers from acrylamide, acrylamidopropyltrimethylammonium chloride, 2-amidopropylacrylamide sulfonate and dimethylaminopropylamine (polyquaternium-43); oligomers or polymers, producible from quaternary crotonoylbetaines or quaternary crotonoylbetaine esters. In an embodiment, the cleansing composition comprises an amphoteric or zwitterionic latex hairstyling polymer. In an embodiment, the hairstyling polymer is selected from the group consisting of: polyquaternium-47, octylacrylamide/acrylate/butylaminoethyl methacrylate copolymers, and mixtures thereof.

The hairstyling formulation may comprise an anionic hairstyling polymer. In an embodiment, the anionic hairstyling polymer is selected from the group consisting of: acrylates copolymers of two or more monomers of (meth)acrylic acid or one of their simple esters (e.g. Balance^{®} CR from Akzo Nobel); acrylates/hydroxyesters acrylates copolymers including those being copolymers of butyl acrylate, methyl methacrylate, methacrylic acid, ethyl acrylate and hydroxyethyl methacrylate (e.g. Acudyne™ 1000 from Dow Personal Care); terpolymers of acrylic acid, ethyl acrylate, and N-tert-butylacrylamide; crosslinked or uncrosslinked vinyl acetate/crotonic acid copolymers; terpolymers of tert-butylacrylate, ethyl acrylate and methacrylic acid; sodium polystyrenesulfonate; copolymers of vinyl acetate, crotonic acid and vinyl propionate; copolymers of vinyl acetate, crotonic acid and vinyl neodecanoate; aminomethylpropanol/acrylate copolymers; copolymers of vinylpyrrolidone and at least one further monomer selected from among acrylic acid, methacrylic acid, acrylic acid esters and methacrylic acid esters; copolymers of methyl vinyl ether and maleic acid monoalkyl esters; aminomethylpropanol salts of copolymers of allyl methacrylate and at least one further monomer selected from among acrylic acid, methacrylic acid, acrylic acid esters and methacrylic acid esters; crosslinked copolymers of ethyl acrylate and methacrylic acid; copolymers of vinyl acetate, mono-n-butyl maleate and isobornyl acrylate; copolymers of two or more monomers selected from among acrylic acid, methacrylic acid, acrylic acid esters and methacrylic acid esters, copolymers of octylacrylamide and at least one monomer selected from among acrylic acid, methacrylic acid, acrylic acid esters and methacrylic acid esters; polyesters of diglycol, cyclohexanedimethanol, isophthalic acid and sulfoisophthalic acid; polyurethanes; and copolymers of polyurethane and acrylates. e.g. polyurethane-14/AMP-acrylates polymer blend (e.g. DynamX^{®} from Akzo Nobel). Suitable polyester polymers include polyester-5 polymers, for example AQ^{®} 48 Ultra Polymer, (diglycol/CHDM/isophthalates/SIP copolymer [a copolymer of diethylene glycol, 1,4-cyclohexanedimethanol and the simple esters of isophthalic acid and sulfoisophthalic acid]), AQ^{®} 55 S, and AQ^{®} 38 S, all from Eastman Chemical Company. Also suitable is a polyvinylmethacrylic acid/maleic acid copolymer (Omnirez^{®} 2000 from ISP). Anionic latex hairstyling polymers are also suitable. In an embodiment, the anionic hairstyling polymer is selected from the group consisting of: polyurethane-1 (e.g. Luviset^{®} P.U.R. from BASF), polyurethane-14/AMP-acrylates copolymer blend (e.g. DynamX^{®} from Akzo Nobel), acrylates copolymers of two or more monomers of (meth)acrylic acid or one of their simple esters (e.g. Balance^{®} CR from Akzo Nobel), and mixtures thereof. In an embodiment, the anionic hairstyling polymer is is polyurethane-1.

The cleansing composition may comprise a non-ionic hairstyling polymer. In an embodiment, the cleansing composition comprises a non-ionic hairstyling polymer, wherein the non-ionic hairstyling polymer is a homo- or copolymer that is formed from at least one of the following monomers: vinylpyrrolidone, vinylcaprolactam, vinyl esters such as, for example, vinyl acetate, vinyl alcohol, acrylamide, methacrylamide, alkyl- and dialkylacrylamide, alkyl- and dialkylmethacrylamide, alkyl acrylate, alkyl methacrylate, propylene glycol or ethylene glycol, where the alkyl groups in these monomers may be C-1 to C-7 alkyl groups or C-1 to C-3 alkyl groups. In an embodiment, the cleansing composition comprises a homopolymer selected from the group consisting of: vinylcaprolactam, vinylpyrrolidone, N-vinylformamide and mixtures thereof. In an embodiment, the non-ionic hairstyling polymer is selected from the group consisting of: copolymers of vinylpyrrolidone and vinyl acetate, terpolymers of vinylpyrrolidone, vinyl acetate and vinyl propionate, polyacrylamides; polyvinyl alcohols as well as polyethylene glycol/polypropylene glycol copolymers; and mixtures thereof. In an embodiment, the non-ionic hairstyling polymer is selected from the group consisting of: polyvinylpyrrolidone/dimethylaminopropylaminoacrylates copolymer (Aquaflex^{®} SF 40 from ISP); isobutylene ethylmaleinimide/hydroxy ethylmaleinimide copolymer (Aquaflex^{®} FX 64 from ISP); vinylcaprolactam/polyvinylpyrrolidone/dimethylaminoethyl methacrylate copolymer (Advantage^{®} from ISP); and mixtures thereof. Non-ionic latex hairstyling polymers are also suitable. In an embodiment, the non-ionic hairstyling polymer is selected from the group consisting of: polyvinylpyrrolidone (K90, 85, 80, 60, 30), polyvinylpyrrolidone/vinyl acetate copolymers (PVP/VA 64), terpolymers of vinylpyrrolidone, methacrylamide and vinylimidazole (e.g. Luviset^{®} Clear from BASF), and mixtures thereof. In an embodiment, the non-ionic hairstyling polymer is selected from the group consisting of: PVP K 60, 30, and PVP/VA 37/64. In an embodiment, the non-ionic hairstyling polymer is selected from the group consisting of: PVP K60 and PVP/VA 37/64.

In an embodiment, the cleansing composition comprises an anionic latex hairstyling polymer. In an embodiment, the anionic latex hairstyling polymer is a urethane-based polymer, for example polyurethane-34 (Baycusan® from Bayer). Polyurethane-34 is described in EP2105127A1. In an embodiment, the hairstyling polymer is the latex hairstyling polymer polyurethane-34.

In an embodiment, the anionic hairstyling polymer and/or cationic hairstyling polymer is present in neutralized or partially neutralized form. In an embodiment, the cleansing composition comprises a neutralising agent, and wherein the neutralising agent is selected from the group consisting of: potassium hydroxide, sodium hydroxide, triisopropanolamine (TIPA), 2-aminobutanol, 2-aminomethyl propanol (AMP), aminoethylpropandiol, dimethyl stearamine (Armeen 18 D), sodium silicate, tetrahydroxypropyl ethylenediamine (Neutrol^{®} TE), ammonia (NH₃), triethanolamine, trimethylamine (Tris Amino Ultra), aminomethylpropandiol (AMPD) and mixtures thereof. In an embodiment, the neutralising agent is 2-aminomethyl propanol.

The cleansing composition is substantially dry. In an embodiment, the cleansing composition is anhydrous. In an embodiment, the cleansing composition is in particulate or powder form. In an embodiment, cleansing composition has a d₅₀ particle size of less than 10 about micron, or less than about 5 micron, or less than about 4 micron, or less than about 2 micron. A d₅₀ particle size means the maximum diameter of 50% of the particles, for example a d₅₀ particle size of 2 micron means 50% of the particles have a diameter of less than 2 micron. Diameter here means the longest dimension of the particle.

An embodiment relates to a cleansing cloth for cleansing hair, wherein the cleansing cloth comprises a substrate comprising a fibrous material, and wherein the cleansing cloth comprises a cleansing composition, wherein the cleansing composition comprises at least about 80% absorber compound, and wherein the cleansing cloth is substantially dry and the cleansing composition is substantially dry, wherein the cleansing cloth comprises from about 0.21 to about 0.42 g/decimetre of the cleansing composition, and wherein the absorber compound is a starch compound and wherein the starch compound is aluminium starch octenylsuccinate, wherein the cleansing composition is substantially free of any volatile organic compound, and wherein the cloth has a maximum length, wherein the maximum length is the longest dimension, from about 20 cm to about 45 cm, and wherein the cleansing composition is substantially free of: castor oil.

### 2nd aspect: Pouch

The second aspect relates to a pouch comprising the cleansing cloth according to the first aspect. The pouch is air- and water-tight or capable of being closed such that when in a closed state, the pouch is air- and water-tight. In an embodiment, the pouch is in the form of a flexible plastics pocket being substantially flat. In an embodiment, the pouch is transparent or translucent. the pouch is in the form selected from the group consisting of: a flexible plastics pocket being substantially flat and a substantially inflexible container comprising at least about 75% plastics or at least about 75% metal. In an embodiment, the pouch comprises the cleansing cloth, wherein the cleansing cloth is substantially dry. The details disclosed herein in relation to the other aspects, particularly the first aspect, are also applicable to the second aspect.

### 3^{rd} aspect: Method

The third aspect relates to a method for cleansing hair, comprising: applying the cleansing cloth, according to the first aspect, to hair; wherein the cleansing composition is applied to substantially dry hair and wherein the method does not comprise wetting the hair. In an embodiment, the method comprises laying the cleansing cloth onto a head of hair and rubbing the hair with the cloth. The details disclosed herein in relation to the other aspects, particularly the first aspect, are also applicable to the third aspect.

### 4th aspect: Use

The fourth aspect relates to the use of the cleansing cloth, according to the first aspect, for cleansing hair. The details disclosed herein in relation to the other aspects, particularly the first aspect, are also applicable to the fourth aspect.

### 5th aspect: Process

The fifth aspect relates to a process for creating a cleansing cloth, wherein the process comprises providing a substrate comprising a fibrous material and then subsequently applying a formulation onto the substrate, such that the cleansing cloth comprises from about 0.2 to about 0.5 g/decimetre of the cleansing composition as described in the first aspect. In an embodiment, the process comprises providing an aerosol dry shampoo product and then subsequently spraying the shampoo product such that a formulation that is ejected from the shampoo product adhere to the substrate and then subsequently allowing the formulation to dry. In an embodiment, the formulation, once dried onto the substrate, is the cleansing composition. In an embodiment, the shampoo product is a dry shampoo are packaged as an aerosol with a propellant in tin-plated steel or aluminium cans. In an embodiment, the shampoo product is a dry shampoo not packaged as an aerosol or spray product. In an embodiment, the shampoo product is a dry shampoo packaged as an aerosol, wherein the formulation in the aerosol can comprises at least 5% absorber compound, and from about 30% to about 50% alcohol, and from about 40% to about 60% propellant. The details disclosed herein in relation to the other aspects, particularly the first aspect, are also applicable to the fifth aspect.

### 6^{th} Aspect: Kit

The sixth aspect relates to a kit comprising: (a) a substrate comprising comprising a fibrous material, wherein the substrate is substantially dry and is substantially free of absorber compound; and (b) an aerosol dry shampoo product comprising a formulation, wherein the formulation in the aerosol dry shampoo product comprises: at least 5% absorber compound, from about 30% to about 50% alcohol, and from about 40% to about 60% propellant. In an embodiment, the absorber compound is a tapioca starch. The details disclosed herein in relation to the other aspects, particularly the first aspect, are also applicable to the sixth aspect.

### EXAMPLES

The following examples further describe and demonstrate embodiments within the scope of the present invention. The examples are given solely for the purpose of illustration and are not to be construed as limitations of the present invention.

### Test 1: Comparison of different cleansing compositions and their adhesion to the substrate (molleton)

This is to test the efficacy of different cleansing compositions. Different aerosol dry shampoo products are evenly sprayed for 10 seconds onto a substrate, allowed to dry and weighed. The substrate used was the same for each: a 1 decimetre square (i.e 10 cm x 10 cm) of molleton being substantially flat and having two faces. Only one face of the substrate is sprayed with the aerosol dry shampoo product. The removal of the cleansing cloth from the pouch and also the subsequent use of the cleansing cloth are simulated and the cleansing cloth weighed after each. The data resultant from this test are depicted in Table 1. The amount of cleansing composition (grams) used in cleansing hair as calculated in Table 1 correlates with the availability of the absorber compound to cleanse the hair.

**Table 1: Comparison of different cleansing compositions and their adhesion to the substrate (molleton)**

| # | Type of starch compound | Weight of (molleton) substrate (g/dm²) | Amount (grams) of cleansing composition on substrate | | | Cleansing composition used up in cleansing hair* | Score |
|---|---|---|---|---|---|---|---|
| | | | Directly after application onto substrate | After 1 x shaking out (A) | After 8x shaking out (B) | | |
| 1 | Aluminium octenylsucci nate | 1.61 | 0.347 g | Remaining on the substrate = 0.208 g; Loss = 40.1% | Remaining on the substrate = 0.015 i.e. 4.32% | 0.193 g i.e. 55.6% | 1 |
| 2 | Aluminium octenylsucci nate | 1.66 | 0.131 g | Remaining on the substrate = 0.091 g; Loss = 30.0% | Remaining on the substrate = 0.039 g i.e. 29.8% | 0.052 g i.e. 39.7% | 9 |
| 3 | Oryza Sativa rice | 1.63 | 0.833 g | Remaining on the substrate = 0.556 g; Loss = 33.3% | Remaining on the substrate = 0.077 g i.e. 9.2% | 0.479 g i.e. 57% | 1 |
| 4 | Corn starch modified | 1.63 | 0.753 g | Remaining on the substrate = 0.487 g; Loss = 35.0% | Remaining on the substrate = 0.05 g i.e. 6.64% | 0.437 g i.e. 58% | 2 |
| 5 | Dimethylim iazolidinone rice | 1.65 | 0.210 g | Remaining on the substrate = 0.210 g; Loss =∼0% | Remaining on the cloth = 0.18 g i.e. 85%. | 0.02 g i.e. 9.5% | 8 |
| 6 | Oryza sativa rice | 1.64 | 0.981 g | Remaining on the substrate = 0.380 g; Loss = 61.2% | Remaining on the substrate = 0.083 g i.e. 8% | 0.3 g i.e. 30.6% | 4 |
| 7 | Aluminium octenylsucci | 1.67 | 0.319 g | Remaining on the | Remaining on the | 0.075 g i.e. 23% | 10 |
| | nate | | | substrate = 0.271 g; Loss = 15.0% | substrate = 0.196 g i.e. 61.4% | | |

| | | | | | | | |
|---|---|---|---|---|---|---|---|
| KEY: # = Aerosol dry shampoo product tested; 1 = Frottee by Swiss O Par; 2 = Bamboo Style by Alterna; 3 = Cotton Fresh by Schauma; 4 = Dry Shampoo, Mid Brown by Lee Stafford; 5 = Morning After by Umberto Giannini; 6 = Dry Shampoo clean & classic by Batiste; 7 = Day 2 Freshness Dry Shampoo by Toni & Guy; A = simulation of removing cleansing cloth from the pouch; B = simulation of using the cleansing cloth to cleanse hair; * calculated as the amount (grams) of cleansing composition lost during the procedure (B) and what percentage that is of the total cleansing composition on the substrate directly after its application onto the substrate; Score = a scale of 1 to 10 was used for the appraisal wherein 10 is the worst possible score and 1 the best. | | | | | | | |

Conclusions from the data in Table 1: For products 1 to 4, about 30-40% of the cleansing composition is lost after one shake out. For product 5, very little cleansing composition is lost from the substrate at any stage, which suggests it is firmly adhered to the substrate and thus less able to cleanse hair in view of adherence of sebum and other hair 'dirt'. For product 7, little cleansing composition is lost after one shake out but significantly more is lost after the subsequent 8x shake out. The opposite is true of product 6. Products 1 and 3 received the highest score. Product 1 is very easy to apply to the substrate in an optimal amount. When sprayed on the cloth, products 1 and 3 both provide a visible, white layer of cleansing composition on the substrate - for product 1, the layer is relatively thin and for product 3, somewhat thicker. When product 4 is sprayed on the substrate, the layer of cleansing composition was brown in colour. For product 6, the layer of cleansing composition is quite thick on the substrate. For product 2, there is no visible cleansing composition on the substrate.

### Test 2: Comparison of aerosol dry shampoo product and cleansing cloth comprising cleansing composition

This is to test the preference of users (assessors) of an aerosol dry shampoo product and of a cleansing cloth. Assessors use an aerosol dry shampoo product (Frottee by Swiss O Par) by spraying it directly onto on one half of their head of hair and using it in the fashion advised on pack. The cleansing cloth is created by spraying the same aerosol dry shampoo product onto a substrate (molleton). The assessors use the cleansing cloth on the other half of their head of hair. Following both applications, assessors evaluate their experiences and provide feedback on specific criteria vis-à-vis their hair post cleansing. The data resultant from this test are depicted in Table 2.

**Table 2: Comparison of aerosol dry shampoo product and cleansing cloth comprising cleansing composition**

| Assessor | 1 | 2 | 3 | 4 | Mean |
|---|---|---|---|---|---|
| Hair colour | reddish-blonde | reddish brown | dark brown | red | |
| Hair structure/type | curly/dry | straight/oily | straight/oily | straight/oily | |

| Appraisal of use of Aerosol Dry Shampoo Product: | | | | | |
|---|---|---|---|---|---|
| Feel of hair | 3 | 9 | 9 | 9 | 7.5 |
| Refreshment of hair | 2 | 2 | 9 | 5 | 4.5 |
| Removal of oiliness | 1 | 2 | 9 | 4 | 4 |
| Hair volume | 3 | 3 | 8 | 5 | 4.75 |
| Visibility of residues | 9 | 9 | 10 | 8 | 9 |

| Appraisal of use of Cleansing Cloth: | | | | | |
|---|---|---|---|---|---|
| Feel of hair | 2 | 4 | 3 | 2 | 2.75 |
| Refreshment of hair | 2 | 2 | 2 | 3 | 2.25 |
| Removal of oiliness | 1 | 2 | 2 | 4 | 2.25 |
| Hair volume | 3 | 3 | 3 | 5 | 3.5 |
| Visibility of residues | 6 | 5 | 3 | 3 | 4.25 |

| | | | | | |
|---|---|---|---|---|---|
| KEY: A scale of 1 to 10 was used for the appraisal wherein 10 is the worst possible score and 1 the best. | | | | | |

Conclusions from the data in Table 2: The cleansing cloth scored, on average across the assessors, the best in every category. The blonde-haired assessor did not more readily tolerate the residues left in view of the cleansing composition being white in colour. Particularly dramatic favourability for the cleansing cloth was in view of the lack of visibility of residues after use and the feel of the hair following use.

### Test 3: Comparison of different substrate fibrous materials

This is to test the efficacy of various substrate fibrous materials. The aerosol dry shampoo product Frottee by Swiss O Par was sprayed for 15 seconds onto a substrate, allowed to dry and weighed. The substrate used was the same size for each: a 1 decimetre square (i.e 10 cm x 10 cm), being substantially flat and having two faces. Only one face of the substrate is sprayed with the aerosol dry shampoo product. The removal of the cleansing cloth from the pouch and also the subsequent use of the cleansing cloth are simulated and the cleansing cloth weighed after each. The same aerosol dry shampoo product was employed for all substrates. The data resultant from this test are depicted in Table 3.

**Table 3: Comparison of different substrate fibrous materials (the same aerosol dry shampoo product i.e. same cleansing composition was utilised)**

| # | Weight of (molleton) substrate (g/dm²) | Amount (grams) of cleansing composition on substrate | | | Cleansing composition (grams) used in cleansing hair* | Score |
|---|---|---|---|---|---|---|
| | | Directly after application onto substrate | After 1x shaking out (A) | After 8x shaking out (B) | | |
| 1 | 1.64 | 4.037 g | Remaining on the substrate = 0.758 g; Loss = 81.2% | Remaining on the substrate = 0.093 g | 0.665 g | 1 |
| 2 | 0.16 | 1.056 g | Remaining on the substrate = 0.328 g; Loss = 68.9% | Remaining on the substrate = 0.058 g | 0.270 g | 5 |
| 3 | 0.15 | 1.147 g | Remaining on the substrate = 0.331 g; Loss = 71.1 % | Remaining on the substrate = 0.048 g | 0.293 g | 4 |
| 4 | 2.84 | 1.237 g | Remaining on the substrate = 0.275 g; Loss = 77.7% | Remaining on the substrate = 0.046 g | 0.230 g | 6 |
| 5 | 0.55 | 1.118 g | Remaining on the substrate = 0.231 g; Loss = 79.3% | Remaining on the substrate = 0.065 g | 0.167 g | 8 |
| 6 | 1.99 | 1.128 g | Remaining on the substrate = 0.15 g; Loss = 86.7% | Remaining on the substrate = 0.037 g | 0.113 g | 10 |
| 7 | 1.33 | 1.718 g | Remaining on the substrate = 0.254 g; Loss = 85.2% | Remaining on the substrate = 0.070 g | 0.184 g | 7 |

| | | | | | | |
|---|---|---|---|---|---|---|
| KEY: # = Fibrous material tested; 1 = Molleton made from cotton and roughened on both sides; 2 = Non-woven hydrophilic; 3 = Non-woven lipophilic; 4 = Microfibre cloth; 5 = Paper towel; 6 = Linen fabric, plain (aka tabby) weave; 7 = Cambric cotton, plain (aka tabby) weave; A = simulation of removing cleansing cloth from the pouch; B = simulation of using the cleansing cloth to cleanse hair; * calculated as the amount (grams) of cleansing composition lost during the procedure (B) and what percentage that is of the total cleansing composition on the substrate directly after its application onto the substrate. | | | | | | |

Conclusions from the data in Table 3: The most preferred substrates were those made from molleton and non-woven.

### Test 4: Comparison of different amounts of cleansing compositions on the substrate

This is a test to compare the effect of different amounts of cleansing composition on the substrate in terms of grams per decimetre. The aerosol dry shampoo product Frottee by Swiss O Par was sprayed onto a substrate, allowed to dry and weighed. The substrate used was the same size for each: a 1 decimetre square (i.e 10 cm x 10 cm), molleton, being substantially flat and having two faces. Only one face of the substrate is sprayed with the aerosol dry shampoo product. The removal of the cleansing cloth from the pouch and also the subsequent use of the cleansing cloth are simulated and the cleansing cloth weighed after each. The same aerosol dry shampoo product was employed for all substrates. The data resultant from this test are depicted in Table 4.

**Table 4: Comparison of different amounts of cleansing compositions on the substrate (the same molleton substrate fibrous material, each cleansing cloth being 1 decimetre in size, was utilised)**

| # | Weight of (molleton) cloth (g/dm²) | Amount of cleansing composition (grams) on substrate | | | Cleansing composition (grams) used in cleansing hair* | Score |
|---|---|---|---|---|---|---|
| | | Directly after application onto substrate | After 1x shaking out (A) | After 8x shaking out (B) | | |
| | | 0.123 g. | Remaining on the substrate = 0.088 g; Loss = 28.46%. Almost no dust. | Remaining on the substrate = 0.013 g i.e. 10.57% | | |
| **1.** | 1.61 | Layer of cleansing composition barely visible. | | | 0.075 g i.e. 60.98% | 6 |
| | | 0.229 g. | Remaining on the substrate = 0.152 g; Loss = 33.62%. Very little dust. | Remaining on the substrate = 0.04 g i.e. 17% | | |
| **2.** | 1.63 | Layer of cleansing composition visible. | | | 0.117 g i.e. 51.09% | 1 |
| | | 0.347 g. | Remaining on the substrate = 0.208 g; Loss = 40.06%. Minimal dust. | Remaining on the substrate = 0.015 g i.e. 4.32% | | |
| **3.** | 1.63 | Very thin layer of white cleansing composition visible. | | | 0.193 g i.e. 55.62% | 1 |
| | | 0.483 g. | Remaining on the substrate = 0.246 g; Loss = 49.07%. Dusty. | Remaining on the substrate = 0.018 g i.e. 1.1% | | |
| **4.** | 1.64 | Structure of substrate still visible. | | | 0.228 g i.e. 47.21% | 3 |
| | | 0.562 g. | Remaining on the substrate = 0.255 g; Loss = 54.63%. Significant dust. | Remaining on the substrate = 0.032 g i.e. 5.69% | | |
| **5.** | 1.63 | Structure of substrate barely visible. | | | 0.223 g i.e. 39.68% | 6 |
| | | 0.654 g. | Remaining on the substrate = 0.322 g; Loss = 50.76%. Very significant dust. | Remaining on the substrate = 0.042 g i.e. 6.42% | | |
| **6.** | 1.60 | Almost only white layer of cleansing composition (i.e. very little substrate visible). | | | 0.28 g i.e. 42.81% | 6 |
| | | 0.757 g | Remaining on the substrate = 0.352 g; Loss = 53.5%. Extremely dusty. | Remaining on the substrate = 0.037 g i.e. 4.89% | | |
| **7.** | 1.67 | Only white layer of cleansing composition visible (i.e. no substrate visible). | | | 0.315 g i.e. 41.61% | 8 |
| | | 0.820 g. | Remaining on the substrate = 0.398 g; Loss = 51.46%. Extremely dusty. | Remaining on the substrate = 0.04 g i.e. 4.87%. | | |
| **8.** | 1.62 | Thick, white layer of cleansing composition and no substrate visable. | | | 0.358 g i.e. 43.55% | 10 |
| KEY: # = Experiment number; A = simulation of removing cleansing cloth from the pouch; B = simulation of using the cleansing cloth to cleanse hair; * calculated as the amount (grams) of cleansing composition lost during the procedure (B) and what percentage that is of the total cleansing composition on the substrate directly after its application onto the substrate. | | | | | | |

Conclusions from the data in Table 4: Experiments 2, 3 and 4 were found to have an acceptable performance in view of the amount of cleansing composition on the substrate.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A cleansing cloth for cleansing hair, wherein the cleansing cloth comprises a substrate comprising a fibrous material, and wherein the cleansing cloth comprises a cleansing composition,
wherein the cleansing composition comprises at least about 20% absorber compound, and wherein the cleansing cloth is substantially dry and the cleansing composition is substantially dry, wherein the cleansing cloth comprises from about 0.2 to about 0.5 g/decimetre of the cleansing composition.

2. The cleansing cloth of claim 1, wherein the absorber compound is selected from the group consisting of: oryza sativa (rice) starch, dimethylimidazolidinone rice starch, aluminium starch octenylsuccinate, corn starch, modified corn starch, zea mays starch, solanum tuberosum starch, tapioca starch, and mixtures thereof.

3. The cleansing cloth according to any of the preceding claims, wherein the absorber compound is a starch compound and wherein the starch compound is aluminium starch octenylsuccinate.

4. The cleansing cloth according to any of the preceding claims, wherein the cleansing composition is substantially free of any volatile organic compound.

5. The cleansing cloth according to any of the preceding claims, wherein the cleansing composition is in particulate or powder form.

6. The cleansing cloth according to any of the preceding claims, wherein the cloth has a maximum length, wherein the maximum length is the longest dimension, of about 10 to about 60 cm, or from about 20 cm to about 45 cm, or from about 30 cm to about 40 cm.

7. The cleansing cloth according to any of the preceding claims, wherein the cleansing cloth is in a shape complementary to and capable of receiving a member selected from the group consisting of: a human hand or human a finger, a comb, a hair brush, a straightening or curling implement, and combinations thereof; or wherein the cloth is a glove for a human hand or human finger.

8. The cleansing cloth according to any of the preceding claims, wherein the cleansing cloth is substantially flat and wherein the cleansing cloth has an area (length multiplied by width) of from about 100 cm² to about 3600 cm².

9. The cleansing cloth according to any of the preceding claims, wherein both the cleansing cloth and the absorber compound exhibit a colour substantially corresponding to the colour of human hair.

10. The cleansing cloth according to any of the preceding claims, wherein the cleansing composition further comprises an additive selected from the group consisting of: glitter, particles, a preservative, a dye or pigment, skin or hair conditioning agent, pH adjuster, buffering agent, fragrance, panthenol, calendula, retinyl palmitate, bromelain, calcium panthotenate, amodimethicone, cyclopentasiloxane, silicone compound, mica, titanium dioxide, iron oxide, ascorbic acid, aminomethyl propanol, adipic acid, disodium phosphate, methyl paraben, propyl paraben, DMDM hydantoin, diazolidinyl urea, geraniol, amyl cinnamal, hexyl cinnamal, lemon grass oil, ascorbyl palmitate, and mixtures thereof.

11. A pouch comprising the cleansing cloth of any of the preceding claims, wherein the pouch is air- and water-tight or capable of being closed such that when in a closed state, the pouch is air- and water-tight.

12. The pouch according to claim 11, wherein the pouch is in the form selected from the group consisting of: a flexible plastics pocket being substantially flat and a substantially inflexible container comprising at least about 75% plastics or at least about 75% metal.

13. A method for cleansing hair, comprising: applying the cleansing cloth, according to any of claims 1 to 10, to hair; wherein the cleansing composition is applied to substantially dry hair and wherein the method does not comprise wetting the hair.

14. Use of the cleansing cloth, according to any of claims 1 to 10, for cleansing hair.

15. A process for creating a cleansing cloth, wherein the process comprises providing a substrate comprising a fibrous material and then subsequently applying a formulation onto the substrate, such that the cleansing cloth comprises from about 0.2 to about 0.5 g/decimetre of the cleansing composition as described in any of claims 1 to 10.
